# EUROPEAN PATENT APPLICATION

(11) **EP 3 339 444 A1**
(43) Date of publication of application: **27.06.2018**
(21) Application number: 16382631.6
(22) Date of filing: 20.12.2016
(51) Int. Cl.: C12P 21/00

(54) **METHOD FOR OBTAINING A GLYCOPROTEIN WITH AN INCREASED PERCENTAGE OF AFUCOSYLATED GLYCANS**

(71) Applicant: Mabxience Research, S.L., 28050 Madrid (ES)
(72) Inventor: BES, Cedric, 28001 Madrid (ES); SÁNCHEZ DE MELO, Iván, 24007 Leon (ES); REAL FERNÁNDEZ, Rebeca, 24010 Trobajo del Camino (ES); SZEKRENYES, Akos, 24001 Leon (ES)
(74) Representative: Ponti & Partners, S.L.P

(57) **Abstract**

The present invention relates to a method for obtaining a glycoprotein with a glycoform composition comprising an increased percentage of afucosylated glycans relative to an identical method performed without arginine which comprises: (i) culturing cells expressing said glycoprotein under conditions adequate for the expression of said glycoprotein in a cell culture medium comprising arginine in a concentration range of between about 1.5 and about 10 g/L; and (ii) recovering and purifying said glycoprotein.

## Description

### FIELD OF THE INVENTION

The present invention relates to glycoproteins. In particular, the present invention relates to a method for obtaining a glycoprotein with a glycoform composition comprising an increased percentage of afucosylated glycans.

### BACKGROUND OF THE INVENTION

Monoclonal antibodies (mAbs) of the IgG type are an important class of therapeutic proteins used for the treatment of cancer, autoimmune, and infectious diseases. IgG antibodies are composed of two heavy chains and two light chains that are associated to form three distinct protein domains, including two variable Fab domains and a constant (crystallizable) Fc domain linked by a flexible hinge region. The Fab domains are responsible for antigen binding, while the Fc domain is engaged in Fc receptor-mediated effector functions, such as antibody-dependent cellular cytotoxicity (ADCC) and complement-dependent cytotoxicity (CDC). X-ray crystallographic and NMR structural studies indicate that the Fc glycans are sandwiched between the two CH2/CH3 subdomains and have multiple noncovalent interactions with the Fc domains. It is also known that the attachment of different Fc glycans can have distinct impact on the Fc domain conformations, implicating an important role of glycosylation in maintaining an appropriate Fc domain structures for interactions with respective Fc receptors associated with antibody's effector functions.

It has been further demonstrated that the fine structures of Fc N-glycans are important determinants of the pro- and anti-inflammatory activities of antibodies. For example, the lack of the core fucose, as well as the attachment of a bisecting GlcNAc moiety, dramatically enhances the affinity of antibody for the FcyIIIa receptor (FcyRIIIa), which is responsible for the antibody-dependent cellular cytotoxicity (ADCC). Thus, low-fucose content mAbs are sought out for improved in vivo anticancer efficacy.

Managing glycosylation profile is a key issue in the development of new biological products. ADCC (Antibody dependent cellular cytotoxicity) and CDC (complement dependent cytotoxicity) play a key role in the action mechanism of therapeutic monoclonal antibodies. Those mechanisms are completely related to the glycosylation profile, having special impact the afucosylation and galactosylation levels on these activities. It is well known that higher afucosylation profile increases the binding to FC receptors, hence ADCC potency is improved. Galactosylation is well described as modulator of CDC potency.

The impact of glycosylation on the biological functions and therapeutic outcome of IgG antibodies has stimulated tremendous interest in developing methods to control antibody's glycosylation. One approach is to control the glycosylation profiles during production through glycan biosynthetic pathway engineering in various expression systems, including mammalian, plant, and yeast host cells. This control of glycosylation has resulted in the production of low-fucose or non-fucosylated monoclonal antibodies with improved ADCC activities. But, the glycoforms that can be generated by this approach have been limited, and in most cases, a complete control to a defined homogeneous glycoform is difficult. Naoko Yamane-Ohnuki et al., Production of therapeutic antibodies with controlled fucosylation, mAbs, p. 230-236, 2009, Vol.1 Issue 3, provide a review of the current technologies for production of therapeutic antibodies with control of fucosylation of the Fc N-glycans.

A recent analysis of several therapeutic glycoprotein drugs on the market has indicated significant changes of the glycosylation profiles from different batches produced in different periods. This analysis implicates the challenge in maintaining consistent production of glycoprotein-based drugs and also raises regulatory concerns, as changes of the Fc glycosylation would most likely impact the therapeutic efficacy.

mAbs which are commercially interesting are those involved in the suppression of the immune response, in particular TNF inhibitors such as adalimumab, infliximab, etanercept, certolizumab pegol or golimumab. For example, adalimumab is a TNF inhibiting anti-inflammatory biologic medication. Adalimumab binds to tumor necrosis factor-alpha (TNFα). TNFα normally binds to TNFα receptors, which leads to the inflammatory response of autoimmune diseases. By binding to TNFα, adalimumab reduces this inflammatory response. Because TNFα is also part of the immune system that protects the body from infection, treatment with adalimumab may increase the risk of infections. Adalimumab was the first fully human monoclonal antibody drug approved by the FDA and it is commonly used for treating for rheumatoid arthritis, psoriatic arthritis, ankylosing spondylitis, Crohn's disease, ulcerative colitis, moderate to severe chronic psoriasis, moderate to severe hidradenitis suppurativa, and juvenile idiopathic arthritis.

Due to the great importance of these antibodies for treating an important group of diseases and also their cost it would be necessary to produce antibodies with better stability qualities in order to extend their "shelf life".

Accordingly, there exists a need for a method for obtaining glycoproteins with enhanced stability and half-life.

In this regard, the present inventors have surprisingly found that the addition of arginine to cell cultures in a method for obtaining glycoproteins, in particular antibodies, and more specifically anti-TNF-α antibodies, such as adalimumab, allows to obtain a glycosylation pattern wherein an increased percentage of afucosylated glycans is observed. This is particularly advantageous because a high percentage of afucosylated glycans provides stability and increases the half-life of the glycoproteins, in particular antibodies, and more specifically anti-TNF-α antibodies, such as adalimumab.

### BRIEF DESCRIPTION OF THE DRAWINGS

- **Figure 1:**: VCD and Viability at different arginine concentration as a function of time.
- **Figure 2:**: VCD and Viability at different arginine concentration as a function of time.
- **Figure 3:**: % Afucosylated glycoforms increase obtained in Media_1 Feed_2.
- **Figure 4:**: % Afucosylated glycoforms increase obtained in Media_2 Feed_2.
- **Figure 5:**: VCD and Viability at different arginine concentration as a function of time.
- **Figure 6:**: % Afucosylated glycoforms increase obtained in Media_2 Feed_1 on day 14.
- **Figure 7:**: VCD and Viability at different arginine concentration as a function of time.
- **Figure 8:**: % Afucosylated glycoforms increase obtained in Media_3 Feed_1 on day 14.
- **Figure 9:**: % Afucosylated glycoforms increase obtained in Media_3 Feed_1 on day 16.
- **Figure 10:**: VCD and Viability at different arginine concentration as a function of time.
- **Figure 11:**: % Afucosylated glycoforms increase obtained in Media_3 Feed_3 on day 14.
- **Figure 12:**: VCD and Viability at different arginine concentration as a function of time.
- **Figure 13:**: % Afucosylated glycoforms increase obtained in Media_3 Feed_4 on day 16.
- **Figure 14:**: VCD and Viability at different arginine concentration as a function of time.
- **Figure 15:**: Increased percentage obtained in Media_2 Feed_1 on day 16.

### SUMMARY OF THE INVENTION

The present invention relates to a method for obtaining a glycoprotein with a glycoform composition comprising an increased percentage of afucosylated glycans relative to an identical method performed without arginine.

### DETAILED DESCRIPTION OF THE INVENTION

The present invention relates to a method for obtaining a glycoprotein with a glycoform composition comprising an increased percentage of afucosylated glycans relative to an identical method performed without arginine which comprises: (i) culturing cells expressing said glycoprotein under conditions adequate for the expression of said glycoprotein in a cell culture medium comprising arginine in a concentration range of between about 1.5 and about 10 g/L, in particular between 1.5 and 10 g/L; and (ii) recovering and purifying said glycoprotein.

Thus, the step (i) of the method of the invention comprises culturing cells expressing the glycoprotein of interest under suitable conditions. The suitable host cells for expressing recombinant glycoproteins as disclosed herein are well known in the art and include any of those described herein, preferably a mammalian cell, such as CHO (Chinese Hamster Ovary) cells, lymphocytic cells (e.g., NSO cells which are cells derived from non-secreting murine myeloma) and a variety of other mammalian cells, more preferably being said suitable host cell CHO cells.

The terms "mammalian host cell," "host cell," and "mammalian cell" refer to cell lines derived from mammals that are capable of growth and survival when placed in either monolayer culture or in suspension culture in a medium containing the appropriate nutrients and growth factors. Typically, such cells are capable of expressing and secreting large quantities of a particular glycoprotein of interest into the culture medium. Examples of suitable mammalian host cells include, but are not limited to, Chinese hamster ovary cells/-DHFR (Urlaub and Chasin, Proc. Natl. Acad. Sci. USA, 77:4216 (1980)); dp12CHO cells (EP 307247); monkey kidney CV1 line transformed by SV40 (ATCC CRL 1651); human embryonic kidney line (293 or 293 cells subcloned for growth in suspension culture) (Graham et al., J. Gen Virol., 36:59 (1977)); baby hamster kidney cells (ATCC CCL 10); mouse sertoli cells (TM4) (Mather, Bibl. Reprod., 23:243-251 (1980)); monkey kidney cells (ATCC CCL 70); African green monkey kidney cells (VERO-76) (ATCC CRL-1587); human cervical carcinoma cells (HeLa) (ATCC CCL 2); canine kidney cells (MDCK) (ATCC CCL 34); buffalo rat liver cells (BRL 3A) (ATCC CRL 1442); human lung cells (W138) (ATCC CCL 75); human liver cells (Hep G2 HB 8065); mouse mammary tumor (MMT 060562, ATCC CCL51); TRI cells (Mather et al., Annals N.Y. Acad. Sci., 383:44-68 (1982)); MRC 5 cells; FS4 cells; and a human hepatoma line (Hep G2).

The term "recombinant host cell", as used herein, is intended to refer to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The terms "expression," "express" and "expresses" generally refer to transcription and translation occurring within a host-cell. The level of expression of gene product in a host cell may be determined on the basis of either the amount of corresponding mRNA that is present in the cell or the amount of the protein encoded by the gene. For example, mRNA transcribed from a product gene can be quantitated by northern hybridization. (Sambrook et al., Molecular Cloning: A Laboratory Manual, pp. 7.3-7.57, Cold Spring Harbor Laboratory Press (1989)). A protein encoded by a gene can be quantitated either by assaying for the biological activity of the protein or by employing assays that are independent of such activity, such as, for example, western blotting analysis or radioimmunoassay using antibodies that are capable of reacting with the protein. (Sambrook et al., Molecular Cloning: A Laboratory Manual, pp. 18.1-18.88 Cold Spring Harbor Laboratory Press (1989)). In some embodiments, the terms "expression," "express" and "expresses" are used in reference to a recombinant protein produced by the method of the invention.

The term "glycoprotein" means proteins that contain oligosaccharide chains (glycans) covalently attached to polypeptide side-chains. The carbohydrate is attached to the protein in a cotranslational or post-translational modification. This process is known as glycosylation.

The term "glycoform composition" or distribution as used herein pertains to the quantity or percentage of different glycoforms present in a glycoprotein. The term "glycoform" or "glycovariant" have been used interchangeably herein, and refers to various oligosaccharide entities or moieties linked in their entirety to the Asparagine 297 (as per Kabat numbering) of the human Fc region of the glycoprotein in question, co-translationally or post-translationally within a host cell. The glycan moieties that may be added during protein glycosylation include G0, G1, G2, G0F, G1 F, G2F, M3, M4, M5-8, M3NAG etc. Table I below shows illustrative glycosylations which can be used in the present invention.

**Table I: Representative table of various glycans**

| **Glycan Structure** | **Code** | **Glycan Structure** | **Code** |
|---|---|---|---|
| 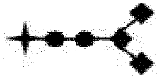 | M3 | 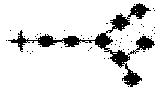 | M6 |
| 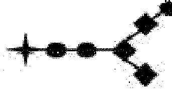 | M3NAG | 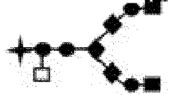 | G₂F |
| 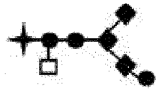 | M3NAGF | 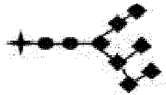 | M7 |
| 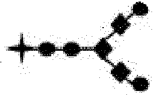 | G₀ | 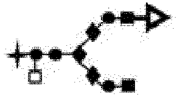 | G₂SF |
| 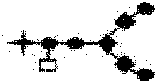 | G₀F | 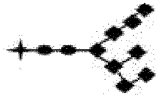 | M8 |
| 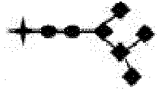 | M5 | 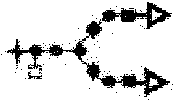 | G₂S₂F |
| 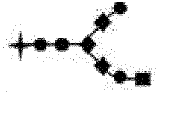 | G_{1A} | 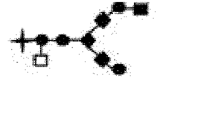 | G_{1A}F |
| 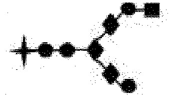 | G_{1B} | 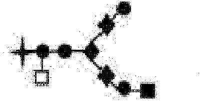 | G_{1B}F |

| | | | |
|---|---|---|---|
| Mannose ◆ N-Acetyl Glucosamine ● Galactose ■ 2-AB Label + Fucose □ sialic acid ▷ | | | |

The term "antibody" refers to molecules which structurally have sugar chains (glycans) added to conserved amino acid residues. In other words, antibodies are glycoproteins. The attached glycans are critically important to the structure and function of the antibody. Among other things the expressed glycans can modulate an antibody's affinity for its corresponding FcR(s). The term "antibody" also refers to an immunoglobulin or antigen-binding fragment thereof. The term includes but is not limited to polyclonal, monoclonal, monospecific, polyspecific, humanized, human, single-chain, chimeric, synthetic, recombinant, hybrid, mutated, grafted, and in vitro generated antibodies. The antibody can include a constant region or a portion thereof, such as those encoded by the kappa, lambda, alpha, gamma, delta, epsilon and mu constant region genes. For example, heavy chain constant regions of the various isotypes can be used including: IgG1, IgG2, IgG3, IgG4, IgM, IgA1, IgA2, IgD and IgE. By way of example, the light chain constant region can be kappa or lambda.

The term "antigen-binding fragment" as used herein may refer to a part of an antibody molecule that comprises amino acids responsible for the specific binding between antibody and antigen. For certain antigens, the antigen-binding fragment may only bind to a part of the antigen. The part of the antigen that is specifically recognized and bound by the antibody is referred as the "epitope" or "antigenic determinant". Antigenic-binding fragments include Fab (Fragment antigen-binding); a F(ab')2 fragment, a bivalent fragment having two Fab fragments linked by a disulfide bridge at the hinge region; Fv fragment; a single chain Fv fragment (scFv), a Fd fragment having the two VH and CH1 domains; dAb and other antibody fragments that retain antigen-binding function. The Fab fragment has a VH-CH1 and VL-CL domains covalently linked by a disulfide bond between the constant regions. The Fv fragment is smaller and has VH and VL domains non-covalently linked. To overcome the tendency of non-covalently linked domains to dissociate, a scFv can be constructed. The scFv contains a flexible polypeptide that links (1) the C-terminus of VH to the N-terminus of VL, or (2) the C-terminus of VL to the N-terminus of VH. These antibody fragments are obtained using conventional techniques known to those skill in the art, and the fragments are evaluated for function in the same manner as are intact antibodies.

Preferably, said anti-TNFα antibody is an antibody against human TNFα. The term "human TNFα" (abbreviated herein as hTNFα, or simply hTNF), as used herein, is intended to refer to a human cytokine that exists as a 17 kD secreted form and a 26 kD membrane associated form, the biologically active form of which is composed of a trimer of noncovalently bound 17 kD molecules. The structure of hTNFα is described further in, for example, Pennica, D., et al. (1984) Nature 312:724-729; Davis, J.M., et al. (1987) Biochemistry 26: 1322-1326; and Jones, E.Y., etal. (1989) Nature 338:225-228. The term human TNFα is intended to include recombinant human TNFα (rhTNFα), which can be prepared by standard recombinant expression methods or purchased commercially (R&D Systems, Catalog No. 210-TA, Minneapolis, MN).

In a particular embodiment, said anti-TNFα antibody is a human antibody, preferably a human anti-hTNFα antibody. The term "human antibody" as used herein, is intended to include antibodies having variable and constant regions derived from human germline immunoglobulin sequences. The human antibodies may include amino acid residues not encoded by human germline immunoglobulin sequences (e.g., mutations introduced by random or site-specific mutagenesis in vitro or by somatic mutation in vivo), for example in the CDRs and in particular CDR3. However, the term "human antibody", as used herein, is not intended to include antibodies in which CDR sequences derived from the germline of another mammalian species, such as a mouse, have been grafted onto human framework sequences.

In a preferred embodiment, the anti-TNFα antibody is adalimumab. Adalimumab, commercialized under the trade name Humira®, is a recombinant human anti-hTNFα IgG1 antibody. This antibody is also known as D2E7. It consists of 1330 amino acids and has a molecular weight of approximately 148 kilodaltons. Adalimumab has been described and claimed in U.S. Pat. No. 6,090,382. The term "adalimumab" is also intended to include the so-called "biosimilar" and "biobetter" versions of the active adalimumab protein present in commercially available Humira®.

As used herein, "biosimilar" (of an approved reference product/biological drug, such as a protein therapeutic, an antibody, etc.) refers to a biologic product that is similar to the reference product based upon data derived from (a) analytical studies that demonstrate that the biological product is highly similar to the reference product notwithstanding minor differences in clinically inactive components; (b) animal studies (including the assessment of toxicity); and/or (c) a clinical study or studies (including the assessment of immunogenicity and pharmacokinetics or pharmacodynamics) that are sufficient to demonstrate safety, purity, and potency in one or more appropriate conditions of use for which the reference product is approved and intended to be used and for which approval is sought for the biological product. In one embodiment, the biosimilar biological product and reference product utilize the same mechanism or mechanisms of action for the condition or conditions of use prescribed, recommended, or suggested in the proposed labelling, but only to the extent the mechanism or mechanisms of action are known for the reference product. In one embodiment, the condition or conditions of use prescribed, recommended, or suggested in the labelling proposed for the biological product have been previously approved for the reference product. In one embodiment, the route of administration, the dosage form, and/or the dose of the biological product are the same as those of the reference product. In one embodiment, the facility in which the biological product is manufactured, processed, packed, or held meets standards designed to assure that the biological product continues to be safe, pure, and potent. The reference product may be approved in at least one of the U.S., Europe, or Japan.

The term "Afucosylated" means the absence of fucose sugar units, wherein fucose is (3*S*,4*R*,5*S*,6*S*)-6-Methyltetrahydro-2*H*-pyran-2,3,4,5-tetraol and may be represented by

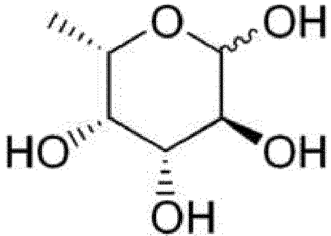

The term "afucosylated (monoclonal) antibody" is a monoclonal antibody engineered so that the oligosaccharides in the Fc region of the antibody do not have any fucose sugar unit. "Afucosylated glycovariants or glycoforms" described herein, refers to glycan moieties wherein fucose is not linked to the non-reducing end of N-acetlyglucosamine except the high mannose forms (for e.g. M3NAG, G₀, Gi_{A}, Gi_{B}, G₂).

As used herein, the term "about" refers to a condition that when so modified is understood to not necessarily be absolute or perfect but would be considered close enough to those of ordinary skill in the art to warrant designating the condition being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, 10, 12, 15, or 20%. Accordingly, the term "about" may mean the indicated value ± 5% of its value, preferably the indicated value ± 2% of this value, most preferably the term "about" means exactly the indicated value (± 0%).

As mentioned above, the step (i) is carried out in suitable conditions for expressing the glycoprotein in a cell culture medium comprising arginine. These conditions include, but are not limited thereto, the concentration of arginine, pH, temperature, further additives.

As used herein, the terms "cell culturing medium" (also called "culture medium", "cell culture media", "tissue culture media") refers to any nutrient solution used for growing cells, e.g., animal or mammalian cells, and which generally provides at least one or more components from the following: an energy source (usually in the form of a carbohydrate such as glucose); one or more of all essential amino acids, and generally the twenty basic amino acids, plus cysteine; vitamins and/or other organic compounds typically required at low concentrations; lipids or free fatty acids; and trace elements, e.g., inorganic compounds or naturally occurring elements that are typically required at very low concentrations, usually in the micromolar range.

The nutrient solution may optionally be supplemented with additional components to optimize growth of cells, such as hormones and other growth factors, e.g., insulin, transferrin, epidermal growth factor, serum, and the like; salts, e.g., calcium, magnesium and phosphate, and buffers, e.g., HEPES; nucleosides and bases, e.g., adenosine, thymidine, hypoxanthine; and protein and tissue hydro lysates, e.g., hydrolyzed animal protein (peptone or peptone mixtures, which can be obtained from animal byproducts, purified gelatin or plant material); antibiotics, e.g., gentamycin; cell protectants or surfactants, polyamines, e.g., putrescine, spermidine or spermine (see e.g., WIPO Publication No. WO 2008/154014) and pyruvate (see e.g. US Patent No. 8053238) depending on the requirements of the cells to be cultured and/or the desired cell culture parameters.

Non-ionic surfactants may also be added to the cell culture medium. Examples of non-ionic surfactants include, but are not limited to, polyvinyl alcohol, polyethylene glycol, and non-ionic block copolymer surfactants. Also included are alkyl poly(ethylene oxide), copolymers of poly(ethylene oxide) and poly(propylene oxide) (EO-PO block copolymers), poly(vinylpyrrolidone), alkyl polyglucosides (such as sucrose monostearate, lauryl diglucoside, or sorbitan monolaureate, octyl glucoside and decyl maltoside), fatty alcohols (cetyl alcohol or olelyl alcohol), or cocamides (cocamide MEA, cocamide DEA and cocamide TEA).

Also included are block copolymers based on ethylene oxide and propylene oxide, also referred to as poly oxypropylene-polyoxy ethylene block copolymers. These molecules are nonionic triblock copolymers having a central hydrophobic chain of polyoxypropylene (poly(propylene oxide)) flanked by two hydrophilic chains of polyoxyethylene (poly(ethylene oxide)). Of particular interest are those having 70 polyoxypropylene units and 30 units of each of the polyoxyethylene chains. In a preferred embodiment the block copolymer is poloxamer 188 (CAS #90003-11-6 with an average molecular weight of 8.4 kd, BASF Chemical, Washington, NJ) which is sold under various brand names such as Pluronic^{®} F68, Kolliphor^{®} P-188, Lutrol^{®}F68, and Lutrol^{®} 188. Such non-ionic surfactants may be added at concentrations up to 5 g/L or more and may be used to maintain cell viability for longer culture durations under ATF perfusion conditions.

Cell culture medium, in certain embodiments, may be serum- free and/or free of products or ingredients of animal origin. Cell culture medium, in certain embodiments, may be chemically defined, where all of the chemical components are known. The term "serum free", means that the medium is essentially free of serum from any mammalian source (e.g. fetal bovine serum [FBS]). By "essentially free" is meant that the cell culture medium comprises between about 0-5% serum, preferably between about 0-1% serum and most preferably between about 0-0.1% serum.

In a particular embodiment, the arginine is present in the cell culture medium in a concentration of 1.5 g/L. In another particular embodiment, the arginine is present in the cell culture medium in a concentration of 2 g/L. In another preferred embodiment, the arginine is present in the cell culture medium in a concentration of 2.5 g/L. In another particular embodiment, the arginine is present in the cell culture medium in a concentration of 3 g/L. In another particular embodiment, the arginine is present in the cell culture medium in a concentration of 3.5 g/L. In another particular embodiment, the arginine is present in the cell culture medium in a concentration of 4 g/L. In another particular embodiment, the arginine is present in the cell culture medium in a concentration of 4.5 g/L. In another particular embodiment, the arginine is present in the cell culture medium in a concentration of 5 g/L. In another particular embodiment, the arginine is present in the cell culture medium in a concentration of 5.5 g/L. In another particular embodiment, the arginine is present in the cell culture medium in a concentration of 6 g/L. In another particular embodiment, the arginine is present in the cell culture medium in a concentration of 6.5 g/L. In another particular embodiment, the arginine is present in the cell culture medium in a concentration of 7 g/L. In another particular embodiment, the arginine is present in the cell culture medium in a concentration of 7.5 g/L. In another particular embodiment, the arginine is present in the cell culture medium in a concentration of 8 g/L. In another particular embodiment, the arginine is present in the cell culture medium in a concentration of 8.5 g/L. In another particular embodiment, the arginine is present in the cell culture medium in a concentration of 9 g/L. In another particular embodiment, the arginine is present in the cell culture medium in a concentration of 9.5 g/L. In another particular embodiment, the arginine is present in the cell culture medium in a concentration of 10 g/l.

Thus, in a particular and preferred embodiment, the arginine is present in the cell culture medium in a concentration of about 4 g/L, in particular 4 g/L.

In a particular embodiment, the cells are cultured at a temperature of about 32ºC to about 38ºC, in particular from 32ºC to 38ºC. In another particular embodiment, the cells are cultured at a temperature of 33ºC. In another particular embodiment, the cells are cultured at a temperature of 34ºC. In another particular embodiment, the cells are cultured at a temperature of 35ºC. In another particular embodiment, the cells are cultured at a temperature of 36ºC. In another particular embodiment, the cells are cultured at a temperature of 37ºC. In another particular embodiment, the cells are cultured at a temperature of 38ºC.

As the person skill in the art will understand, the culture of the cells according to the present invention will be carried out during a suitable time for the production of a glycoprotein with a glycoform composition comprising an increased percentage of afucosylated glycans. In a particular embodiment, the culture is carried out for 7 days. In another particular embodiment, the culture is carried out for 8 days. In another particular embodiment, the culture is carried out for 9 days. In another particular embodiment, the culture is carried out for 10 days. In another particular embodiment, the culture is carried out for 11 days. In another particular embodiment, the culture is carried out for 12 days. In another particular embodiment, the culture is carried out for 13 days. In another particular embodiment, the culture is carried out for 14 days. In another particular embodiment, the culture is carried out for 15 days. In another particular embodiment, the culture is carried out for 16 days. In another particular embodiment, the culture is carried out for 17 days. In another particular embodiment, the culture is carried out for 18 days. In another particular embodiment, the culture is carried out for 19 days. In another particular embodiment, the culture is carried out for 20 days. In a particular embodiment, the culture is cultured at pH from about 6 to about 8.5, in particular at pH from 6 to pH 8.5. In another particular embodiment, the culture is cultured at pH of 6.5. In another particular embodiment, the culture is cultured at pH of 7. In another particular embodiment, the culture is cultured at pH of 7.5. In another particular embodiment, the culture is cultured at pH of 8. In another particular embodiment, the culture is cultured at pH of 8.5.

In a particular embodiment, the cell culture medium additionally comprises about 0.1-5 mg/ml FerCit, in particular 0.1-5 mg/ml FerCit, and/or about 0.1-15 mM ac.3Met, in particular 0.1-15mM ac.3Met.

As shown in the example section, the cell medium composition which can be used is the following:

| Ingredient | mM |
|---|---|
| Arginine | 0.2 - 30.0 |
| Aspartic Acid | 2.0 - 25.0 |
| Asparagine | 2.0 - 10.0 |
| Cystine | 0.02 - 5.0 |
| Glutamine | 0.5 - 5.0 |
| Histidine | 0.2 - 4.0 |
| Hydroxyproline | 0.02 - 4.0 |
| Isoleucine | 0.5 - 10 |
| Lysine | 0.1 - 20 |
| Phenylalanine | 0.02 - 4 |

In another preferred embodiment, after the cell culture of step (i) has been established and before performing step (ii), glucose, cysteine and/or tyrosine are fed to the cell culture. In a preferred embodiment, the cysteine amino acid is added in its oxidized dimer form, as cystine.

Cell cultures can also be supplemented with independent concentrated feeds of particular nutrients which may be difficult to formulate or are quickly depleted in cell cultures. Such nutrients may be amino acids such as tyrosine, cysteine and/or cystine (see e.g., WIPO Publication No. 2012/145682). For example, a concentrated solution of tyrosine may be independently fed to a cell culture grown in a cell culture medium containing tyrosine. A concentrated solution of tyrosine and cystine may also be independently fed to the cell culture being grown in a cell culture medium lacking tyrosine, cystine and/or cysteine. The independent feeds can begin prior to or at the start of the production phase. The independent feeds can be accomplished by fed batch to the cell culture medium on the same or different days as the concentrated feed medium. The independent feeds can also be perfused on the same or different days as the perfused medium. Feed medium may include hormones, growth factors, ions, vitamins, nucleoside, nucleotides, trace elements, amino acids, lipids or glucose. These supplementary components may be added at one time or in series of additions to replenish. Thus feed can be a solution of depleted nutrient(s), mixture of nutrient(s) or a mixture of cell culture medium/feed providing such nutrient(s). In one aspect of the invention, concentrated basal media may be used as a feed while in the other specific commercial feeds may be added to the cell culture medium.

As shown in the example section, the feed medium composition which can be used is the following:

| Ingredient | mM |
|---|---|
| Arginine | 15 - 50.0 |
| Aspartic Acid | 10.0 - 40.0 |
| Asparagine | 2.0 - 60.0 |
| Cystine | 0.02 - 5.0 |
| Glutamine | 0.05 - 10.0 |
| Histidine | 2 - 40.0 |
| Hydroxyproline | 0.1 - 10.0 |
| Isoleucine | 3 - 45 |
| Lysine | 5 - 40 |
| Phenylalanine | 0.1 - 35 |
| Proline | 5.0 - 45 |
| Serine | 10.0 - 60.0 |
| Methionine | 1.0 - 25.0 |
| Threonine | 2.0 - 43.0 |
| Tryptophan | 0.2 - 25.0 |
| Tyrosine | 0.1 - 5.0 |
| Valine | 5.0 - 50.0 |
| Glucose | 50.0 - 500.0 |
| Pyruvic acid | 0.5 - 40.0 |

The method of the present invention is suitable for obtaining a glycoprotein as defined herein. A preferred embodiment of the glycoprotein to be obtained is an antibody. More preferably, said antibody is an anti TNF-α antibody such as adalimumab, infliximab, etanercept, certolizumab pegol or golimumab. Most preferably, said antibody is adalimumab.

According to the method of the present invention a glycoprotein with a glycoform composition comprising an increased percentage of afucosylation is obtained relative to an identical method performed without arginine

The term "percentage of afucosylation" refers to the level of afucosylation in the Fc region in a composition of a glycoprotein. The percentage of afucosylation can be measured by any suitable technique known by the person skilled in the art, i.e., mass spectrometry (MS) and presented as the percentage of afucosylated glycan species (species without the fucose on one Fc domain (minus 1) and on both Fc domains (minus 2) combined) over the entire population of glycoprotein. For example, % afucosylation can be calculated as the percentage of the combined area under the minus 1 fucose peak and minus 2 fucose peak over the total area of all glycan species analyzed by MS, such as determined by an Agilent 6520B TOF Mass Spectrometer. The level of afucosylation can be measured by any other suitable methods known in the art, including without limitation HPLC-Chip Cube MS (Agilent) and reverse phase-HPLC.

In a preferred embodiment, the increased percentage of afucosylated glycans is of at least 60% with respect of the total content of glycoforms. In a particular embodiment, said increased percentage of afucosylated glycans comprises an increased percentage of the B glycoform of at least 15%, an increased percentage of the G0 glycoform of at least 20%, an increased percentage of the G1 glycoform of at least 20% and/or an increased percentage of the afucosylation of at least 30%. In another particular embodiment, said increased percentage of afucosylated glycans comprises an increased percentage of the B glycoform of at least 30%, an increased percentage of the G0 glycoform of at least 30% and/or an increased percentage of the G1 glycoform of at least 30%. The percentage of afucosylated glycans can be measured by any suitable method known by the person skilled in the art such as chromatography methods or mass spectrometry methods. In a preferred embodiment, the percentage of afucosylated glycans is measured as follows: N-linked glycans were released from IgG samples by overnight incubation with N-glycosidase. The released glycans were labeled with a specific fluorophore. The labeled glycans were bound and separated by a chromatography. N-linked glycan structures were assigned to peaks based on comparison to reference structures. The amount of each structure is expressed as the percentage of total peak area. G0, G1, and G2 refer to complex, neutral, biantennary structures with 0, 1, or 2 terminal galactose residues, while G0F, G1 F, are G2F, are the corresponding core fucosylated structures. The level of afucosylation can be also measured by any other suitable method known in the art, including without limitation HPLC-Chip Cube MS (Agilent) and reverse phase-HPLC.

Optionally, if desired the level of afucosylation of a glycoprotein composition might further be increased by methods known in the art, including without limitation, by purification. For example, the fucosylated species in a composition can be enriched by affinity chromatography having resins conjugated with a fucose binding moiety, such as an antibody or lectin specific for fucose, especially fucose present in the 1-6 linkage. See e.g., Kobayashi et al, 2012, J. Biol. Chem. 287:33973-82. In certain other embodiments, the fucosylated species can be enriched and separated from afucosylated species using an anti-fucose specific antibody in an affinity column. Alternatively or additionally, afucosylated species can be separated from fucosylated species based on the differential binding affinity using affinity chromatography.

Overall afucosyation percentage of the glycoform composition of the invention may be calculated as follows:
% Afucosylation: [G0-GlcNac + G0 + G1 + G2] / [GO-GlcNac + G0 + G0F + G1 + G1 F + G2 + G2F]

G0 as used herein refers to protein glycan not containing galactose at the terminal end of the glycan chain.
G0-GlcNac as used herein refers to protein glycan not containing galactose at the terminal end of the glycan chain but containing one GlcNac residue more than the core structure.
G1 as used herein refers to protein glycan containing 1 galactose at the terminal end of the glycan chain.
G2 as used herein refers to protein glycan containing 2 galactose at the terminal end of the glycan chain.
G0F as used herein refers to protein glycan not containing galactose at the terminal end of the glycan chain but containing 1 fucose at the terminal end of the glycan chain.
G1 F as used herein refers to protein glycan containing 1 galactose at the terminal end of the glycan chain and containing 1 fucose at the terminal end of the glycan chain.
G2F as used herein refers to protein glycan containing 1 galactose at the terminal end of the glycan chain and containing 2 fucose at the terminal end of the glycan chain.

The step (ii) of the method of the invention comprises recovering and purifying said glycoprotein. The recombinant glycoprotein of interest can be recovered from the culture medium using techniques which are well established in the art. The glycoprotein of interest is preferably recovered from the culture medium as a secreted polypeptide, although it also may be recovered from host cell lysates.

In an embodiment, the cell culture medium or lysate is centrifuged to remove particulate cell debris. The glycoprotein thereafter is purified from contaminant soluble proteins and polypeptides using a suitable purification procedures. Exemplary purification procedures include, but are not limited to, fractionation on immunoaffinity or ion-exchange columns; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; and protein A Sepharose columns to remove contaminants such as IgG. A protease inhibitor such as phenyl methyl sulfonyl fluoride (PMSF) also may be useful to inhibit proteolytic degradation during purification. One skilled in the art will appreciate that purification methods suitable for the recombinant glycoprotein of interest may require modification to account for changes in the character of the glycoprotein upon expression in recombinant cell culture.

It should be noted that all the previous embodiments can be practiced independently from each other or combined with any other embodiment disclosed herein.

The present invention will be now further illustrated by reference to the following examples which do not intend to limit the scope of the present invention.

### EXAMPLES

### Example 1: Production of glycoprotein in a 15 mL culture

A CHO cell line producing adalimumab was cultured in a commercially available cell culture medium (it is a chemically defined, protein-free and animal origin component-free formulation containing amino acids like glutamine, asparagine and histidine and saccharides like glucose) and fed with Feed Medium and a Cystine/Tyrosine mixture. Arginine was added on day 0 at a concentration of 4 g/L. The amount of glucose was adjusted so as to bring the concentration thereof to about 3-4 g/L over the whole process.

The cells were cultured in AMBR vessels in the cell culture media described above. The vessels were maintained at 36.5ºC, pH 7±0.2, 20% of DO and at 900 rpm of stirrer. They were seeded at 0.5 x 10⁶ cells per ml in a volume of 11 mL. On days 0, 3, and 5, at least 2% of initial culture volume of feed medium and 0.2% of initial culture volume of cystine/tyrosine were added. Day 0 is the day in which the cultures were started, day 1 is the first day thereafter, day 2 is the second day thereafter, etc. Protein titer was determined on days 10, 12, 14 and 16.

Exemplary media composition which can be used is the following:

| Ingredient | mM |
|---|---|
| Arginine | 0.2 - 30.0 |
| Aspartic Acid | 2.0 - 25.0 |
| Asparagine | 2.0 - 10.0 |
| Cystine | 0.02 - 5.0 |
| Glutamine | 0.5 - 5.0 |
| Histidine | 0.2 - 4.0 |
| Hydroxyproline | 0.02 - 4.0 |
| Isoleucine | 0.5 - 10 |
| Lysine | 0.1 - 20 |
| Phenylalanine | 0.02 - 4 |

### Example 2: Production of glycoprotein in a 1 L culture

The cells were cultured in 1 L scale bioreactor in the above described cell culture media. The cultures were maintained at 36.5ºC, pH 7±0.2, 20% of oxygen dissolved (DO) and at 200 rpm of stirrer. They were seeded at 0.5 x 10⁶ cells per ml in a volume of 600mL. On days 0, 3, and 5, at least 2% of initial culture volume of feed medium and 0.2% of initial culture volume of cystine/tyrosine were added.

### Example 3: Effect of Arginine on glycosylation profile

Firstly, experiments were carried out to observe effects of arginine on viable cell density (VCD) and viability. As shown below the addition of 2 g/L and 4 g/L arginine to culture media reduces VCD in a maximum about 2 million cells but does not affect viability. The addition of 8 g/L arginine produces an important reduction on cell growth affecting viability.

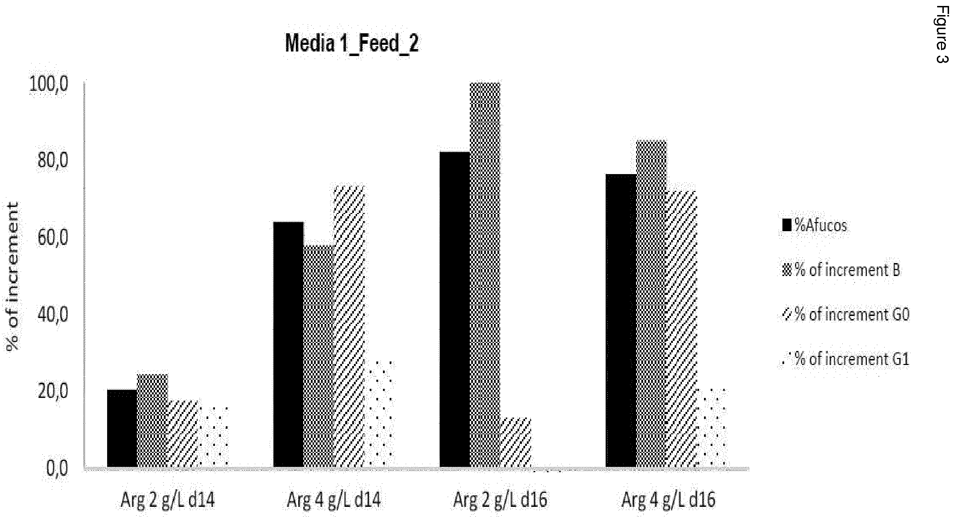

The glycosylation profile was determined with varying amounts of added arginine. As shown on figures 3, 4, 6, 8, 9, 11 and 13, afucosylated glycoforms are increased when 4 g/L arginine is added to the cell medium regardless of the used basal media. G0 and B glycoforms were the main contributors of this effect. The same effect was observed in different production model such as AMBR and 1 L Scale Bioreactor.

### Determination of glycosylation profile and effects of arginine on viable cell density (VCD) and viability.

Here we describe a methodology for modulating several glycoform such as G0, G1, G1 F and G2F which has a very high contribution in the total sum of afucosylation and galatosylation level.

### Material and Methods

A transduced CHO cell line was used with three different culture media (Media 1, 2 and 3) in a fed-batch process with 5 different feeds (Feed 1,2,3,4 and 5) and at two culture scales, AMBR system (15 mL culture) and 1 L scale Bioreactors. Feeding strategy was based on maintaining glucose concentration between 3-4 g/L through the addition of different amount of feeding depending on glucose concentration measurement in culture media.

Process parameters & Conditions AMBR: T: 36.5ºC; Stirrer: 900 rpm; DO: 20%; pH:6.8-7.20; initial density: 0.5x10⁶ cells/mL.
Process parameters & Conditions 1 L: T: 36.5ºC; Stirrer: 200 rpm/240 rpm (day 7); DO: 20%; pH: 6.8-7.20; initial density: 0.5x10⁶ cells/mL.

N-linked glycans were released from IgG samples by overnight incubation with N-glycosidase. The released glycans were labeled with a specific fluorophore. The labeled glycans were bound and separate by a chromatography. N-linked glycan structures were assigned to peaks based on comparison to reference structures. The amount of each structure is expressed as the percentage of total peak area. G0, G1, and G2 refer to complex, neutral, biantennary structures with 0, 1, or 2 terminal galactose residues, while G0F, G1 F, are G2F, are the corresponding core fucosylated structures.

Afucosylation: [GO-GlcNac + G0 + G1 + G2] / [GO-GlcNac + G0 + G0F + G1 + G1 F + G2 + G2F]

### Results

### 1) Doses response curve 0-8 g/L Arg AMBR system: Media_1 Feed_2 and Media_2 Feed_2

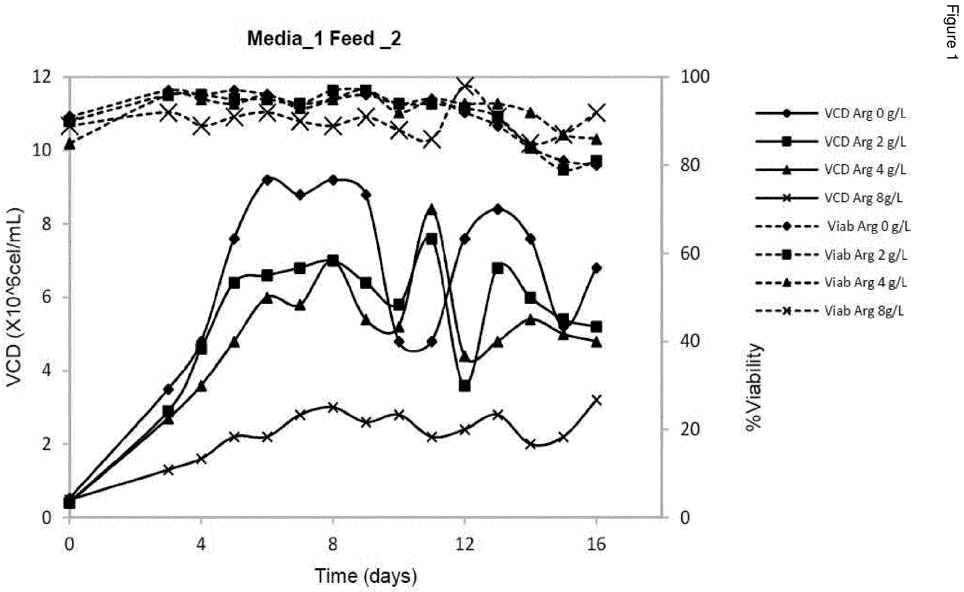

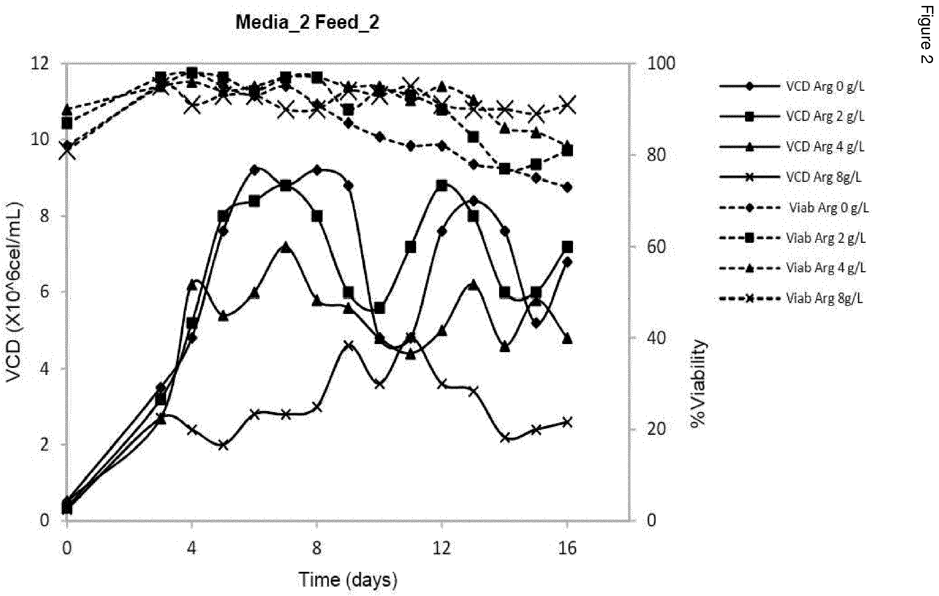

a) VCD-Viability. As shown in figure 1, the addition of arginine to culture media reduces cell growth; the addition of 2 and 4 g/L arginine reduces VCD in a maximum about 2 million cells but do nott affect viability. The addition of 8 g/L arginine produces an important reduction on cell growth affecting viability. As shown in figure 2, the addition of arginine to culture media from 4 to 8 g/L reduces cell growth; the addition of 2 g/L arginine to media_2 does not affect maximum VCD. The addition of 8 g/L arginine produces an important reduction on cell growth, although this effect is lower than in Media_1.
b) Titer

**Table 2: Protein concentration expressed as grams per litre at different days of fed-batch process. Results show a slightly concentration decrease at arginine concentrations of 2 and 4g/L, and an important decrease at 8g/L.**

| **Days** | **Media**_**1 Arg 0 g/L** | **Media_1Arg 2 g/L** | **Media_1 Arg 4 g/L** | **Media_1 Arg 8g/L** |
|---|---|---|---|---|
| 10 | 2.15 | 2.1 | 1.48 | 0.5 |
| 12 | 2.32 | 2.25 | 1.94 | 0.59 |
| 14 | 2.36 | 2.2 | 2.15 | 0.74 |
| 16 | 2.34 | 2.15 | 2.18 | 1.02 |

**Table 3: Protein concentration expressed as grams per litre on different days of fed-batch process in a media_2 Feed_2. Results show a slightly concentration decrease at arginine concentrations of 2 and 4 g/L, and an important decrease at 8 g/L.**

| **Days** | **Media_2 Arg 0 g/L** | **Media_2Arg 2 g/L** | **Media_2 Arg 4 g/L** | **Media_2 Arg 8 g/L** |
|---|---|---|---|---|
| 10 | 2.35 | 2.04 | 1.58 | 0.57 |
| 12 | 2.73 | 2.31 | 2.04 | 0.74 |
| 14 | 2.39 | 2.25 | 2.15 | 0.94 |
| 16 | 2.32 | 1.98 | 2 | 1.24 |

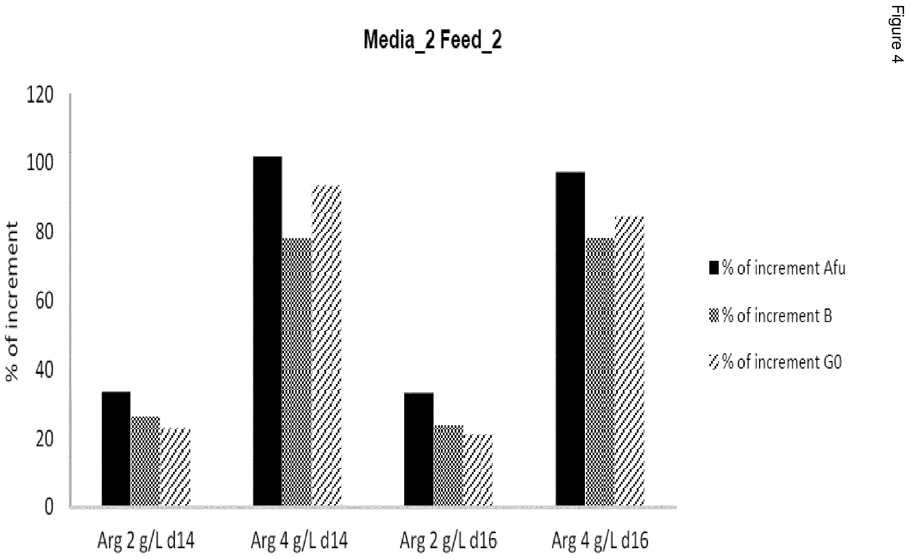

c) Afucosylation/G0/G. As shown in figure 3, the addition of arginine addition to culture media at 2 and 4 g/L increases B, G0 and G1 glycoforms. This effect is seen on days 14 and 16 of the fed-batch culture. As shown in figure 4, the addition of arginine to culture media at 2 and 4 g/L produces an important increase in either afucosylated forms (B and G0 forms). This effect is seen on days 14 and 16 of the fed-batch culture.

### 2) Doses response curve 0-4 g/L Arginine AMBR system: Media_2 Feed_1

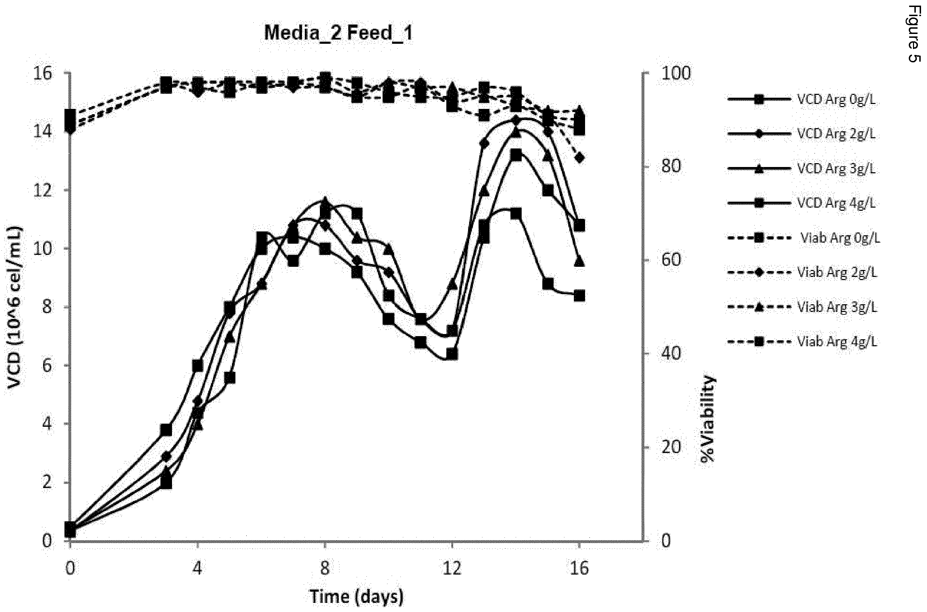

a) VCD-Viability. As shown in figure 5, the addition of arginine addition to culture media does not reduce the maximum cell density reached, although it is reached later as arginine concentration is increased.
b) Titer

**Table 4: Protein concentration expressed as grams per litre on different days of fed-batch process. Results show a progressive concentration decrease as arginine concentration is increased.**

| **Days** | **Media_2 Arg 0 g/L** | **Media_2Arg 2 g/L** | **Media_2 Arg 3 g/L** | **Media_2 Arg 4 g/L** |
|---|---|---|---|---|
| 10 | 3.04 | 2.64 | 2.34 | 2.11 |
| 12 | 4.02 | 3.41 | 3.08 | 2.84 |
| 14 | 4.07 | 3.43 | 3 | 2.76 |
| 16 | 3.84 | 3.23 | 2.65 | 2.63 |

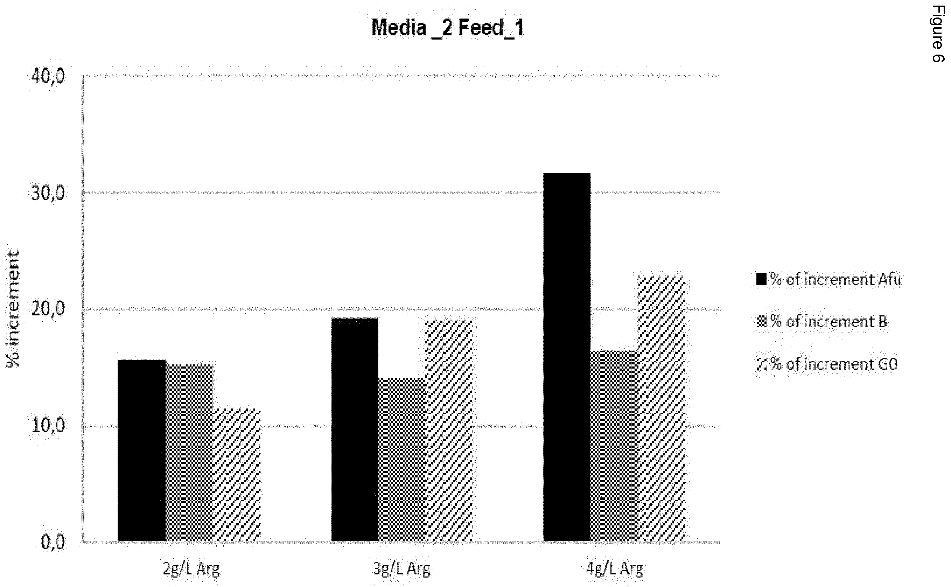

c) Afucosylation/G0/G1. As shown in figure 6, the addition of arginine to the culture media from 2 to 4 g/L produces an increase in the afucosylated level.

### 3) Doses response curve 0-4 g/L Arginine AMBR system: Media_3 Feed_1

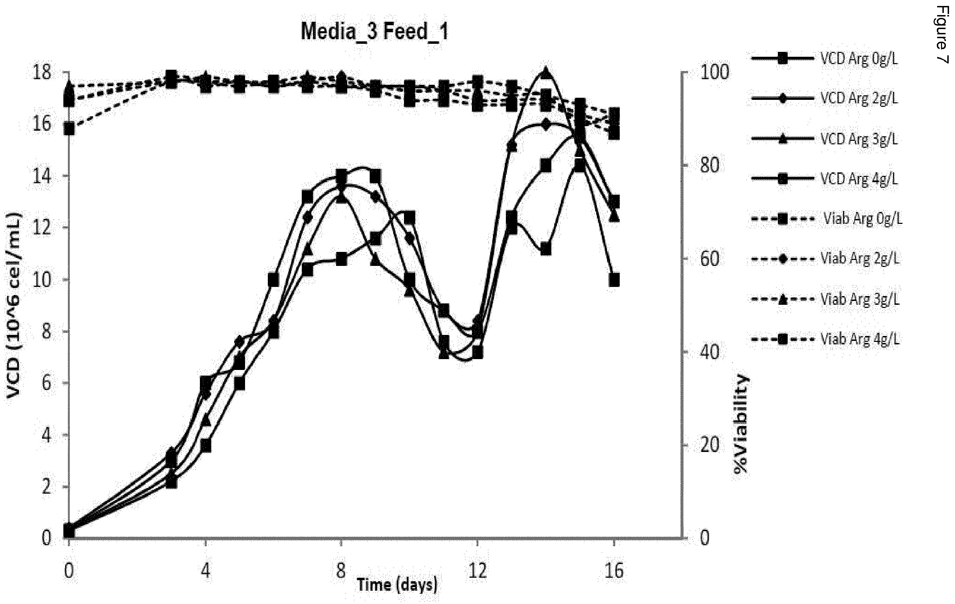

a) VCD-Viability. As shown in figure 7, the addition of arginine to culture media from 2 to 4g/L reduces cell growth; the addition of arginine at 4 g/L seems to delay the maximum cell density reached.
b) Titer

**Table 5: Protein concentration expressed as grams per litre on different days of fed-batch process. Results show a progressive concentration decrease as arginine concentration is increased.**

| **Days** | **Media_3 Arg 0 g/L** | **Media_3Arg 2 g/L** | **Media_3 Arg 3 g/L** | **Media_3 Arg 4g/L** |
|---|---|---|---|---|
| 10 | 2.7 | 2.28 | 2.09 | 2.44 |
| 12 | 3.64 | 3.51 | 3.05 | 2.8 |
| 14 | 3.75 | 3.69 | 3.48 | 3.33 |
| 16 | 3.56 | 3.21 | 3.08 | 2.91 |

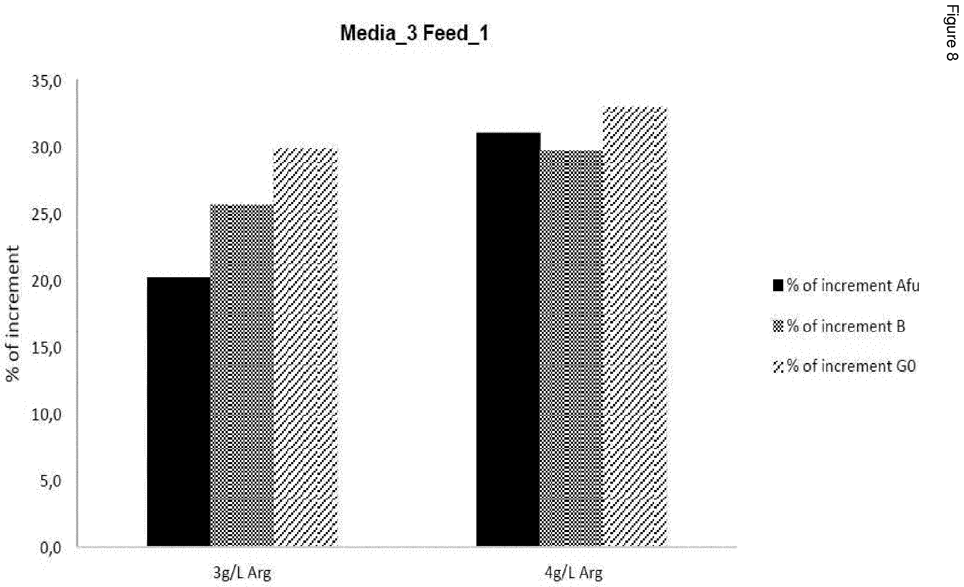

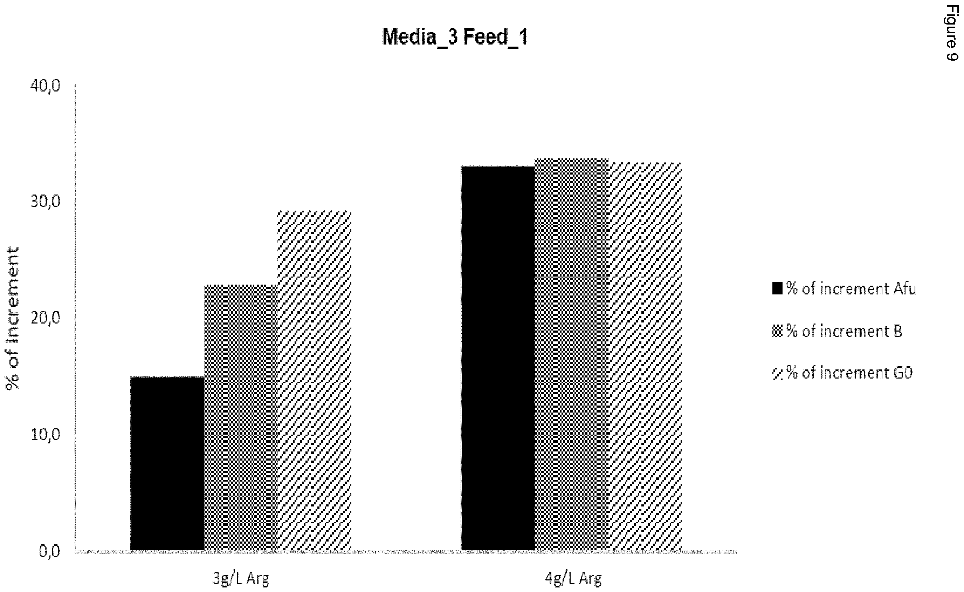

c) Afucosylation/G0/G1. As shown in figure 8, the addition of arginine to culture media from 3 to 4 g/L produces an increment in afucosylated, B and G0 forms on day 14 of fed-batch process. As shown on figure 9, the addition of arginine to culture media from 3 to 4 g/L produces an increment in afucosylated, B and G0 forms on day 16 of fed-batch process.

### 4) Doses response curve 0-4g/L Arginine AMBR system Media_3 Feed_3 AMBR System

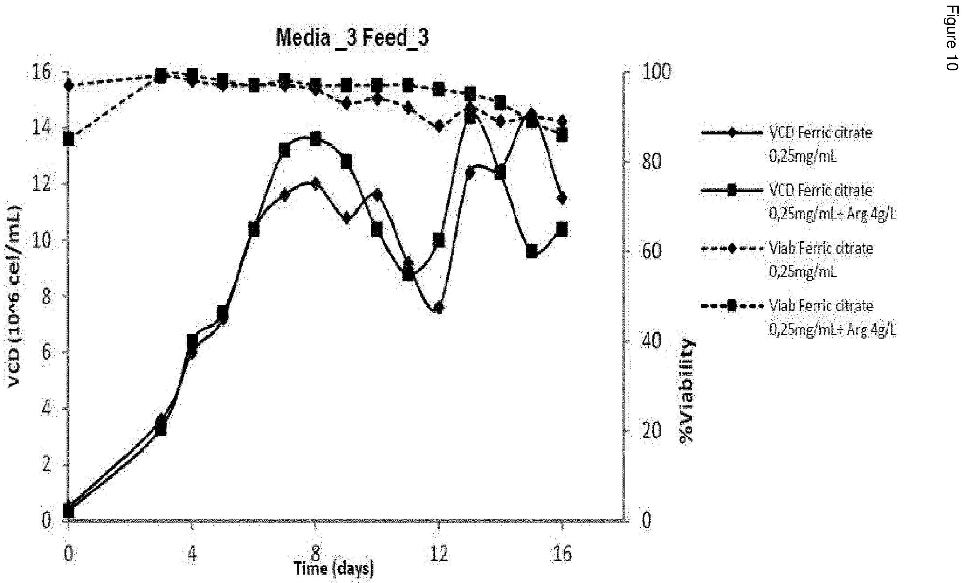

a) VCD-Viability. As shown in figure 10, the addition of arginine to culture media at 4 g/L in combination with ferric citrate does not have a negative effect in cell density.
b) Titer

**Table 6: Protein concentration expressed as grams per litre on different days of fed-batch process.**

| **Days** | **Media_3 Feed_3 Arg 0 g/L** | **Media_3 Feed_3 Arg 4 g/L** |
|---|---|---|
| 10 | 3.28 | 3.02 |
| 12 | 3.62 | 3.34 |
| 14 | 3.85 | 3.33 |
| 16 | 3.62 | 3.12 |

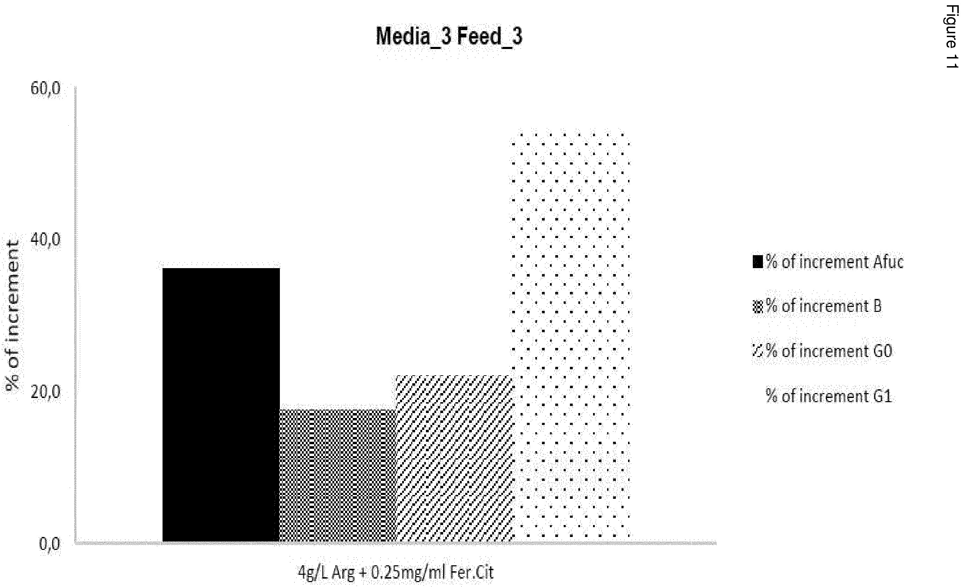

c) Afucosylation/G0/G1. As shown on figure 11, the addition of arginine to culture media at 4 g/L produces an increment in afucosylated, B, G0 and G1 forms on day 14. This increment is higher than 50% for G1 forms.

### 5) Doses response curve 0-4 g/L Arginine AMBR system Media_3 Feed_4 AMBR system

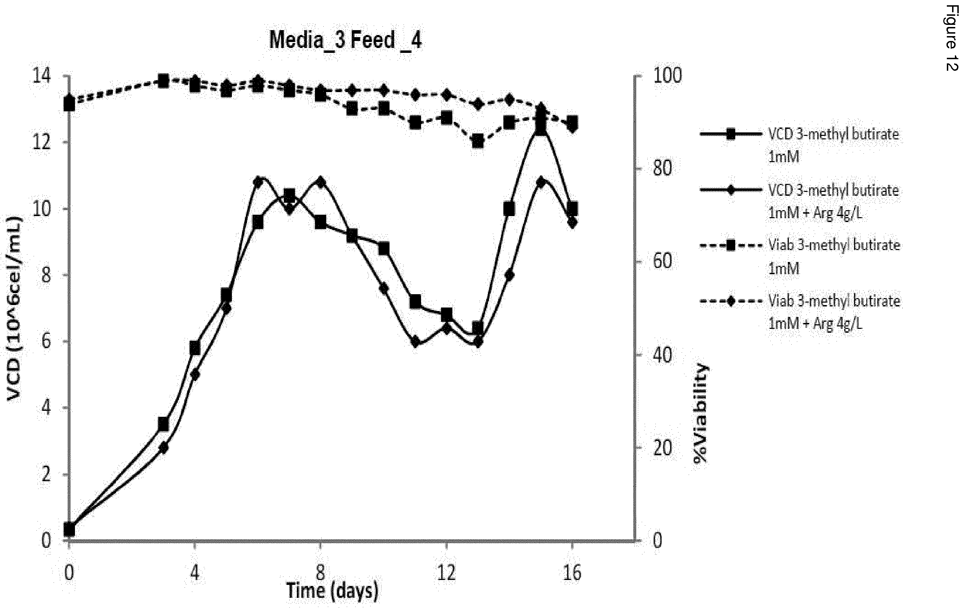

5a) VCD-Viability. As shown in figure 12, the addition of arginine to culture media at 4 g/L in combination with 1 mM 3-methyl butyrate does not have a negative effect in cell density.
b) Titer

**Table 7: Protein concentration expressed as grams per litre on different days of fed-batch process. Results show a concentration decrease on days 10 and 12 of fed-batch process when arginine is not added at 4g/L to culture media, but this effect is not observed on day 16 of fed-batch process.**

| **Days** | **Media_3 Feed_4 Arg 0 g/L** | **Media_3 Feed_4 Arg 4 g/L** |
|---|---|---|
| 10 | 1.19 | 2.2 |
| 12 | 3.03 | 3.42 |
| 14 | 3.59 | 3.87 |
| 16 | 3.32 | 3.28 |

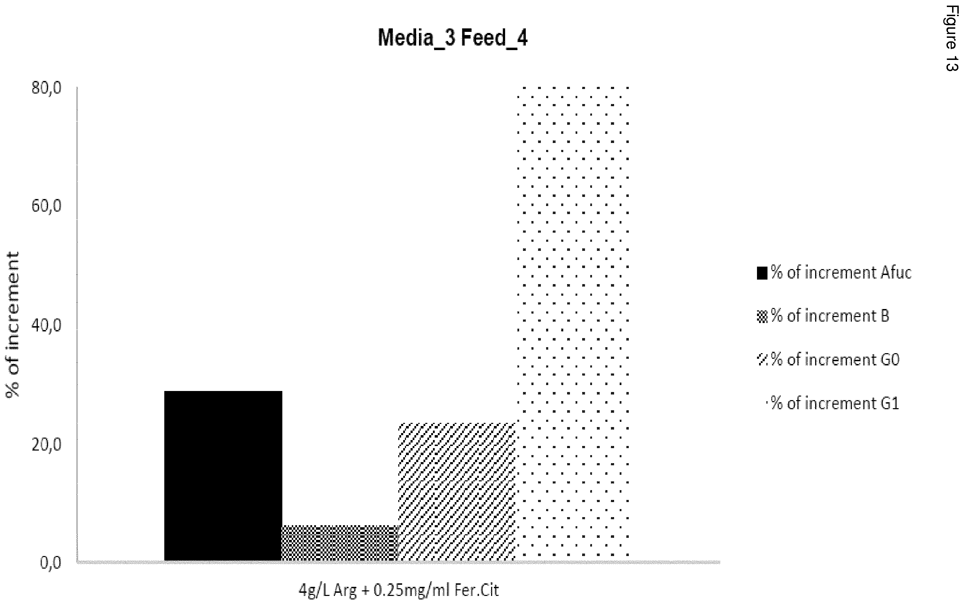

c) Afucosylation/G0/G1. As shown in figure 13, the addition of arginine to culture media at 4g/L produces an increment in afucosylated, B, G0 and G1 forms on day 16 of fed-batch process. This increment is higher than 50% for G1 forms.

### 6) Scale up: Media_2 Feed_1 Liter Bioreactor

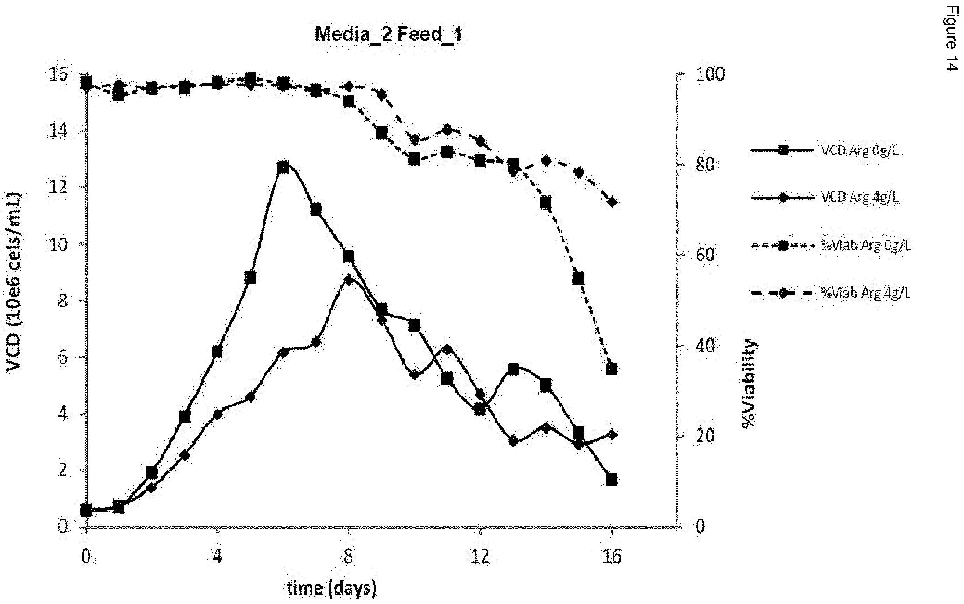

a) VCD-Viability. As shown in figure 14, the addition of arginine to culture media at 4g/L produces a delay and a decrease in the obtained maximum cell density.
b) Titer

**Table 8: Protein concentration expressed as grams per litre on different days of fed-batch process. Results show a concentration decrease when arginine is added at 4 g/L to culture media. This effect is higher on days 10, 12 and 14, but at the end of the process the concentration as obtained is similar to the control condition.**

| **Days** | **Media_2 Feed_1 Arg 0 g/L** | **Media_2 Feed_1 Arg 4 g/L** |
|---|---|---|
| 10 | 1.80 | 1.27 |
| 12 | 2.64 | 1.96 |
| 14 | 2.91 | 2.34 |
| 16 | 2.94 | 2.83 |

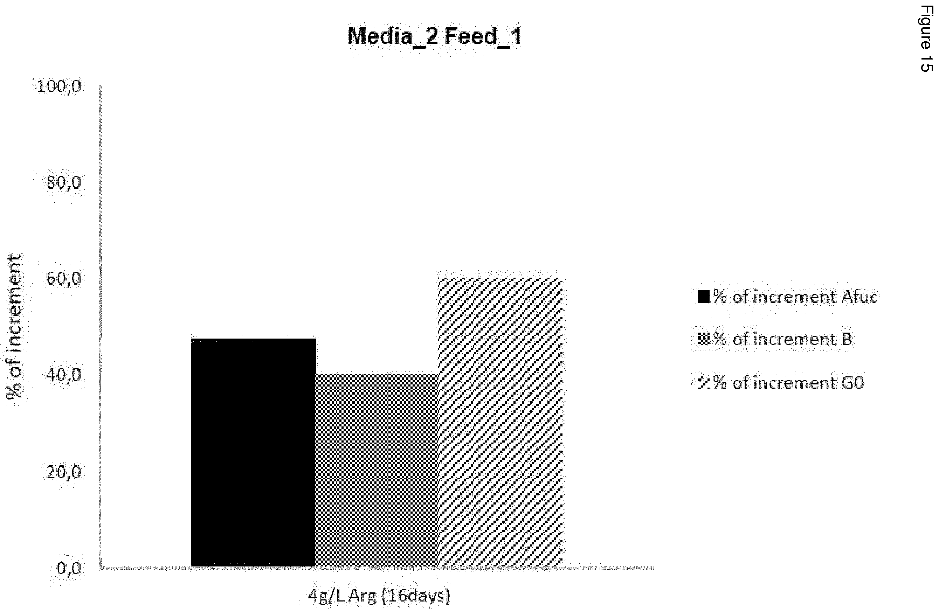

c) Afucosylation/G0/G1. As shown in figure 15, the addition of arginine to culture media at 4 g/L produces an increment in B and G0 forms on day 16 of fed-batch process. This increment is higher than 50% for G0 forms.

### 7) Bioassay table (FCRIII/ADCC)

| **Sample** | **Format** | **Relative EC50 for ADCC activity*** |
|---|---|---|
| 0 gr/L Arg | 1L scale | 79.5 |
| 4 gr/L Arg | | 100.2 |

| | | |
|---|---|---|
| ****Relative data to reference standard Humira® (adalimumab)*** | | |

### Conclusions

Differences in glycosylation pattern were observed when arginine was added to different media culture and feedings. The G0 and G1 glycoforms were exponentially increased with the addition of arginine in either of tested upstream process. The observed change in the glycosylation profile affects the total afucosylation of the antibody and therefore in the functional activity thereof.

## Claims

1. A method for obtaining a glycoprotein with a glycoform composition comprising an increased percentage of afucosylated glycans relative to an identical method performed without arginine which comprises: (i) culturing cells expressing said glycoprotein under conditions adequate for the expression of said glycoprotein in a cell culture medium comprising arginine in a concentration range of between about 1.5 and about 10 g/L; and (ii) recovering and purifying said glycoprotein.

2. The method of claim 1 wherein the arginine is in a concentration of about 4 g/L.

3. The method of claims 1 or 2 wherein glucose, cysteine and/or tyrosine are fed to the cell culture before step (ii).

4. The method of claim 1 to 3, wherein the increased percentage of afucosylated glycans is of at least 60% with respect of the total content of glycoforms.

5. The method of claim 4, wherein said increased percentage of afucosylated glycans comprises an increased percentage of the B glycoform of at least 15%, an increased percentage of the G0 glycoform of at least 20%, an increased percentage of the G1 glycoform of at least 20% and/or an increased percentage of the afucosylation of at least 30%.

6. The method of claim 4, wherein said increased percentage of afucosylated glycans comprises an increased percentage of the B glycoform of at least 30%, an increased percentage of the G0 glycoform of at least 30% and/or an increased percentage of the G1 glycoform of at least 30%.

7. The method of claims 1 to 6, wherein the cells in step (i) are cultured at temperature of about 32ºC to about 38ºC.

8. The method of claims 1 to 7 wherein the culture is carried out for 14 days or for 16 days.

9. The method of claims 1 to 8, wherein the cells are cultured at pH of about 6 to about 8.5.

10. The method of claims 1 to 9, wherein the cell culture medium additionally comprises about 0.1-5 mg/ml FerCit and/or about 0.1-15mM ac.3Met.

11. The method of any of claims 1-10, wherein said cell is a mammalian cell.

12. The method of claim 11, wherein said mammalian cell is a Chinese Hamster Ovarian (CHO) cell.

13. The method of claims 1 to 12, wherein said glycoprotein is an antibody.

14. The method of claim 13, wherein said antibody is an anti TNF-α antibody.

15. The method of claim 13, wherein said antibody is adalimumab.
